# EUROPÄISCHE PATENTSCHRIFT

(11) **EP 1 893 604 B1**
(45) Veröffentlichungstag und Bekanntmachung des Hinweises auf die Patenterteilung: **11.02.2009**
(21) Anmeldenummer: 06761943.7
(22) Anmeldetag: 26.05.2006
(51) Int. Cl.: C07D 401/14

(54) **VERFAHREN ZUR HERSTELLUNG VON N,N-BIS(PYRIDIN-2-YL-METHYL)-1,1-BIS(PYRIDIN-2-YL)-1-AMINOALKAN-VERBINDUNGEN UND DIESE VERBINDUNGEN ENTHALTENDE METALLKOMPLEX-SALZE**
METHOD FOR PRODUCING N,N-BIS(PYRIDIN-2-YL-METHYL)-1,1-BIS(PYRIDIN-2-YL)-1-AMINOALKANE COMPOUNDS AND METAL COMPLEX SALTS CONTAINING THESE COMPOUNDS
PROCEDE POUR PRODUIRE DES COMPOSES N,N-BIS(PYRIDIN-2-YL-METHYL)-1,1-BIS(PYRIDIN-2-YL)-1-AMINO ALCANE ET SELS DE COMPLEXES METALLIQUES CONTENANT CES COMPOSES

(30) Priorität: 01.06.2005 DE 102005024997
(43) Veröffentlichungstag der Anmeldung: 05.03.2008
(73) Patentinhaber: Clariant Produkte (Deutschland) GmbH, 65929 Frankfurt am Main (DE)
(72) Erfinder: DUECKER, Barbara, 55122 Mainz (DE); WESSLING, Michael, 79400 Kandern (DE); NITSCHKE, Nicole, 65760 Eschborn (DE)
(74) Vertreter: Hütter, Klaus
(86) Internationale Anmeldenummer: PCT/EP2006/005023
(87) Internationale Veröffentlichungsnummer: WO 2006/128635

(56) Entgegenhaltungen:
- EP-A2- 0 909 809

## Beschreibung

N,N-Bis(pyridin-2-yl-methyl)-1,1-bis(pyridin-2-yl)-1-aminoalkan-Verbindungen und ein Verfahren zu deren Herstellung sind aus EP 909 809 bekannt. Diese Verbindungen sowie Metallkomplexe, die diese Verbindungen als Liganden enthalten, werden als so genannte Bleichkatalysatoren zusammen mit einer Persauerstoffverbindung in Wasch- oder Reinigungsmitteln eingesetzt um die Temperatur, bei der die Persauerstoffverbindungen den aktiven Sauerstoff freisetzen, herabzusetzen. Die Synthese dieser Verbindungen verläuft gemäß EP 909 809 nach folgendem Reaktionsschema:

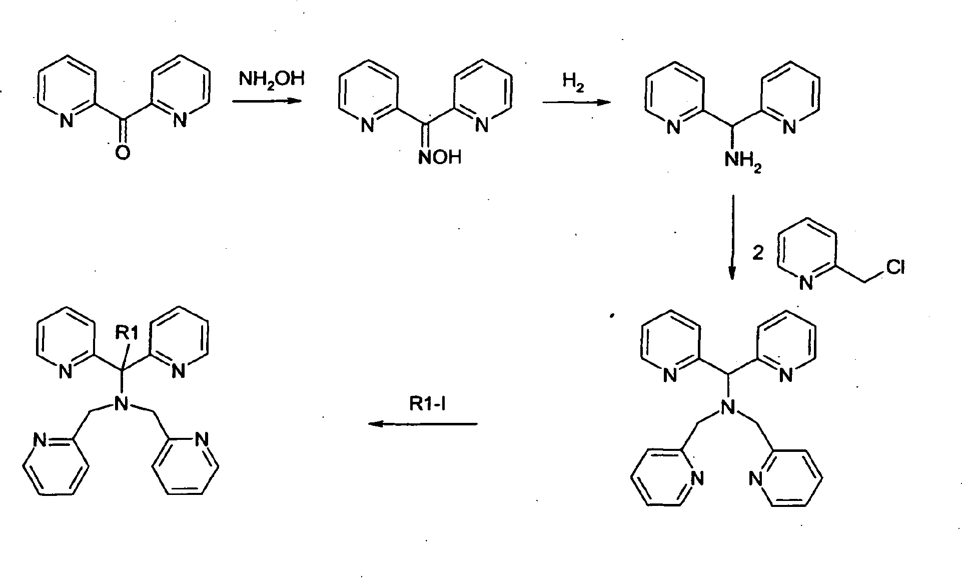

Ausgehend von Dipyridylketon wird durch Umsetzung mit Hydroxylamin das Oxim gebildet, welches anschließend zum Amin reduziert wird.

Die Synthese im ersten Reaktionsschritt erfolgt entweder mit Hydroxylamin in Pyridin oder mit Hydroxylaminhydrochlorid in Ethanol/Wasser unter Zusatz von Natronlauge. Die Isolierung erfolgt durch Neutralisation und Ausfällung. Im zweiten Reaktionsschritt wird das Produkt des ersten Reaktionsschrittes mit Zink und Ammoniak in Ethanol zum Amin reduziert. Die Ausbeuten sind jedoch sehr schwankend. Im dritten Reaktionsschritt wird das primäre Amin mit Picolylchloridhydrochlorid in 5N Natronlauge alkyliert; die Reaktionszeit beträgt 40 h, die Isolierung des Produktes erfolgt durch Fällung mit Perchlorsäure als Perchlorat, welches gewaschen und gegebenenfalls um kristallisiert wird; aus dem gereinigten Salz wird das Amin durch Umsetzung mit Natronlauge freigesetzt, die Isolierung erfolgt durch Extraktion mit Dichlormethan, Trocknen der organischen Phase und Einengen zur Trockne. Im vierten Reaktionsschritt wird frisch freigesetztes Amin in trockenem Tetrahydrofuran mit n-Butyllithium deprotoniert und mit Methyliodid alkyliert; die Mischung wird über Nacht auf -30 - 0°C erwärmt und dann mit Glaubersalz versetzt. Die Isolierung erfolgt durch Einengen, Aufnehmen in Dichlormethan, Waschen, Trocknen und erneutes Einengen; das Rohprodukt wird aus Essigester/Hexan umgefällt. Zur Herstellung der entsprechenden Metallkomplexverbindungen wird der aus den Reaktionsstufen 1 bis 4 erhaltene und umkristallisierte Ligand in Methanol gelöst und mit einer Übergangsmetallverbindung, beispielsweise Eisen(II)chloridtetrahydrat, gelöst in Methanol, umgesetzt. Das Lösungsmittel wird im Vakuum vollständig abgezogen und der harte, fest anhaftende Rückstand umkristallisiert oder ausgerührt. Die erzielen Ausbeuten für die Liganden (Stufe 1 bis 4) liegen im Bereich von 4,9 % bis 5,1 %. Die Ausbeuten für die Komplexe (Stufe 1 bis 5) liegen im Bereich von 3,5 %.

Das beschriebene Syntheseverfahren ist im großtechnischen Maßstab nicht durchführbar; der Lösemitteleinsatz ist hoch, die Isolierung der Produkte ist aus sicherheitstechnischen Gründen problematisch und so technisch nicht durchführbar, die Ausbeuten sind schlecht reproduzierbar, ein Recycling von Lösemitteln unmöglich.

Aufgabe war ein Verfahren zur Herstellung der genannten Verbindungsklasse zu entwickeln, das frei ist von den oben geschilderten Nachteilen.

Gegenstand der Erfindung ist ein Verfahren zur Herstellung von N,N-Bis(pyridin-2-ylmethyl)-1,1-bis(pyridin-2-yl)-1-aminoalkan-Verbindungen der Formel 1 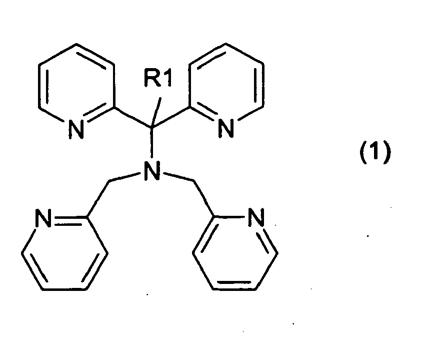 wobei R¹ C₁-C₆-Alkyl, Phenyl oder Benzyl, vorzugsweise Methyl bedeutet. Dieses Verfahren besteht darin, dass man
1) Dipyridylketon mit Hydroxylamin umsetzt, das so erhaltene Oxim ohne Zwischenisolierung zum Amin der Formel 2 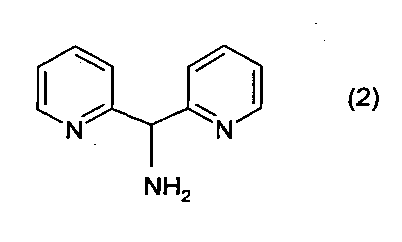 reduziert
2) das Amin der Formel 2 zunächst mit 1 bis 1,5 Moläquivalent Picolylchlorid und dann mit weiteren 1 bis 1,5 Moläquivalenten Picolylchlorid zum Amin der Formel 3 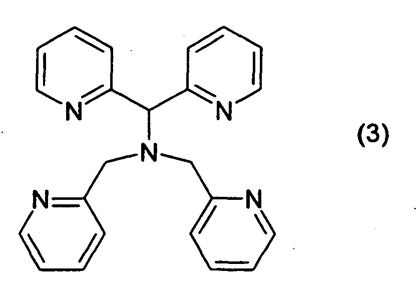 alkyliert,
3) das so erhaltene Amin der Formel 3 durch Zugabe von HBF₄ in das Fluoroborat-Salz überführt und anschließend das Amin der Formel 3 aus diesem Fluoroborat-Salz durch Zugabe einer Base freisetzt, und
4) das Amin der Formel 3 mit einem Alkylhalogenid der Formel R¹-hal alkyliert.

Das erfindungsgemäße Verfahren wird im Einzelnen wie folgt durchgeführt.

### 1. Reaktionsschritt:

Dipyridylketon wird in Methanol vorgelegt. Unter Rühren wird wässrige Hydroxylaminlösung so zugegeben, dass die Mischung sich erwärmt, jedoch nicht zum Sieden kommt. Die Menge an Hydroxylamin beträgt ca. 1,0-1,2 bevorzugt 1,05-1,15 Moläquivalente. Die Mischung wird zum Sieden erhitzt und bei dieser Temperatur bis zur vollständigen Umsetzung gerührt. Die Reaktionszeit beträgt 5 bis 15 h bevorzugt 5 bis 8 h. Anschließend lässt man auf Raumtemperatur abkühlen. Ist diese erreicht wird Palladium auf Kohle als Katalysator zugegeben. Die Menge an Katalysator beträgt ca. 1 bis 5 Gew.-% bezogen auf das eingesetzte Keton. Dann wird unter kräftigem Rühren bei 15 bis 50°C bevorzugt bei 40 bis 50°C Wasserstoff eingeleitet bis die Reduktion vollständig ist. Nach beendeter Reaktion wird die rötliche, klare Lösung vom Katalysator abfiltriert. Zur Isolierung des Produktes wird das Lösemittel im Vakuum vollständig abdestilliert.

Nach dem erfindungsgemäßen Verfahren wird das Zwischenprodukt der Formel 2 in Ausbeuten > 85 %, bevorzugt in Ausbeuten von 88 bis 91 % und hoher Reinheit (> 90 % Gehalt nach NMR) erhalten.

### 2. Reaktionsschritt

Das Produkt der ersten Stufe wird bei Raumtemperatur mit 5N Natronlauge versetzt. Unter Kühlung wird eine zuvor hergestellte Lösung aus Picolylchloridhydrochlorid in Wasser so zugegeben, dass die Innentemperatur nur schwach ansteigt. Die Menge an Natronlauge beträgt 1,5-2,5 bevorzugt 2,0-2,2 Moläquivalente, die Menge an Picolylchlorid beträgt 1,0-1,5, bevorzugt 1,1-1,2 Moläquivalente. Es wird bei Raumtemperatur nachgerührt. Die Reaktionszeit beträgt ca. 1 bis 8 h bevorzugt 2-5h. Dann werden auf die gleiche Weise nacheinander nochmals Natronlauge und Picolylchloridhydrochloridlösung. Die Menge an Natronlauge beträgt 1,5-2,5 bevorzugt 2,0-2,2 Moläquivalente, die Menge an Picolylchlorid beträgt 1,0-1,5 bevorzugt 1,1-1,2 Moläquivalente. Anschließend wird bei Raumtemperatur mehrere Tage kräftig gerührt. Die Reaktionszeit beträgt ca. 2 - 4, bevorzugt 3 Tage. Zur Isolierung wird mit Dichlormethan extrahiert. Nach Einengen wird das Rohprodukt als zähflüssiges Öl erhalten. Die Reinigung erfolgt durch Fällung mit HBF4-Lösung (50 %ig in Wasser) und gleichzeitiger Zugabe von gesättigter Kochsalzlösung unter Kühlung. Der Niederschlag wird abfiltriert, gewaschen und getrocknet.

Man erhält hierbei ein Fluoroboratsalz, das zirka 60 % der Verbindung der Formel 3 enthält.

Aus dem Salz kann das Amin wie folgt wieder freigesetzt werden:

Das Salz wird in Dichlormethan bei Raumtemperatur fein suspendiert. Dann wird 5N Natronlauge zugegeben, und die Mischung kräftig gerührt. Die Menge an Natronlauge beträgt 5-10 bevorzugt 6-7 Moläquivalente bezogen auf das eingesetzte primäre Amin. Die Reaktionszeit beträgt 0,5 bis 2 h bevorzugt 0,5 bis 1 h.

Zur Aufarbeitung werden die Phasen getrennt, die wässrige Phase mit Dichlormethan extrahiert, die vereinigten organischen Phasen über Na₂SO₄ gründlich getrocknet, und das Lösemittel abdestilliert.

Das Produkt der Formel 3 wird nach diesem erfindungsgemäßen Verfahren in Ausbeuten > 50 % bevorzugt in Ausbeuten von 55 bis 65 % und hoher Reinheit (>90 % nach HPLC) erhalten.

### 3. Reaktionsschritt:

Frisch freigesetztes Amin der Formel 3 wird in THF gelöst und die Lösung unter Argon auf -60 bis -80°C, bevorzugt -75 bis -65°C gebracht. Bei dieser Temperatur wird gekühltes n-BuLi so zugetropft, dass die Temperatur nicht zu stark ansteigt. Die Menge an n-BuLi beträgt 1,0-1,2 bevorzugt 1,05-1,15 Moläquivalente. Die Innentemperatur sollte -50°C bevorzugt -65°C nicht überschreiten. Nach beendeter Zugabe wird einige Zeit nachgerührt. Die Reaktionszeit beträgt 30-90, bevorzugt, 30-60 Minuten. Nach Ablauf dieser Zeit wird TMEDA (N,N,N,N-Tetramethylethylendiamin) so zudosiert, dass die Innentemperatur nicht mehr als 5 bis 10°C steigt, und es wird wenige Minuten nachgerührt. Die Menge an TMEDA beträgt 1,0-1,2 bevorzugt 1,05-1,15 Moläquivalente. Nach analogem Verfahren wird dann das Alkyliodid zugegeben. Die Menge an Alkyliodid beträgt 1,0-1,5 bevorzugt 1,1-1,2 Moläquivalente. Anschließend wird die Mischung unter Rühren zunächst auf -40 bis -20, bevorzugt, -30°C, dann auf -5 bis +5°C erwärmt. Die Reaktionszeit beträgt 5-10 bevorzugt 7 bis 9 h. Anschließend wird bei 0°C Glaubersalz hinzugefügt und 15-60 bevorzugt 30-45 Minuten gerührt. Die Menge an Glaubersalz beträgt 0,1-0,2 bevorzugt 0,15-0,17 Moläquivalente.

Der gebildete Niederschlag wird abfiltriert und mit kaltem THF gewaschen. Bei Bedarf kann er im Vakuum getrocknet werden.

Das feuchte Rohprodukt wird im 2-Phasengemisch aus Dichlormethan und 5N Natronlauge kräftig gerührt. Die Reaktionszeit beträgt 5 bis 15 bevorzugt 8 bis 10 h. Anschließend werden die Phasen getrennt, die wässrige Phase mit Dichlormethan extrahiert, die vereinigten organischen Phasen gut mit Natriumsulfat getrocknet und vollständig eingeengt.

Das Produkt der Formel 1 wird nach diesem erfindungsgemäßen Verfahren in Ausbeuten > 60 % bevorzugt in Ausbeuten von 65 bis 70 % und hoher Reinheit (ca. 95 % nach NMR/HPLC) erhalten.

Das Verfahren, wie in EP 909 809 beschrieben, ist großtechnisch in vielen Punkten nicht realisierbar, die benötigte Katalysatormenge ist zu groß, die verwendeten Lösemittelgemische sind schwer oder gar nicht wieder zu trennen, die Masse an verwendeten organischen Lösemitteln ist extrem hoch, die Fällung mit Perchlorat ist aus sicherheitstechnischen Gründen nicht möglich.

Die Herstellung nach dem erfindungsgemäßen Verfahren erfordert demgegenüber einen separaten Reaktionsschritt weniger, benötigt nur vier verschiedene organische Lösemittel anstatt sieben. Die Masse an verwendetem organischem Lösemittel wurde auf zirka 10 % reduziert. Die Menge an kostspieligem Katalysator wurde ebenfalls auf 10 % reduziert. Das Verfahren ist im Vergleich zum Stand der Technik technisch durchführbar.

Die so hergestellten Verbindungen der Formel 1 können unmittelbar als Bleichaktivatoren verwendet werden. Alternativ hierzu kann man diese Verbindungen der Formel durch Reaktion mit Metallsalzen in Metallkomplexsalze überführen, die die Verbindungen der Formel 1 als Liganden enthalten. Hierzu kann man im Einzelnen wie folgt vorgehen:

Eine Verbindung der Formel 1 wird in Methanol suspendiert vorgelegt. Bei Raumtemperatur wird festes Metallsalz zugegeben und nachgerührt. Als Metallsalze kommen hier Fe-, Mn-, Cr-, Co-Salze, vorzugsweise Fe-Salze in Frage.

Die so erhaltenen Metallkomplexsalze eignen sich ebenfalls als Bleichkatalysatoren. Die Reaktionszeit beträgt 2 bis 5 bevorzugt 3 bis 4 h. Anschließend wird ein Teil des Lösemittels im Vakuum abdestilliert. Die Menge an verbleibendem Lösemittel liegt bei 10-20, bevorzugt bei 10-15 % der eingesetzten Menge. Die erhaltene Produktsuspension wird mit Essigsäureethylacetat versetzt und kräftig ausgerührt. Die Dauer des Ausrührens liegt bei 1 bis 5 bevorzugt 2 bis 3 h. Dann wird der Niederschlag abfiltriert, mit weiterem Essigsäureethylacetat gewaschen und im Vakuum getrocknet.

Das Produkt wird nach diesem erfindungsgemäßen Verfahren in Ausbeuten > 90 % bevorzugt in Ausbeuten von 92 bis 94 % und hoher Reinheit (ca. 94 % nach HPLC) erhalten.

Nachfolgende Beispiele sollen die Erfindung näher erläutern ohne sie darauf einzuschränken.

### 1. Stufe:

Es wurden 1,37 I Methanol (1085 g) vorgelegt und 200 g (1,09 mol) Dipyridylketon eingetragen. Dann wurden unter Rühren 65,6 ml (73,5 g) Hydroxylaminlösung (1,14 mol Hydroxylamin) so zugegeben, dass die Mischung sich erwärmte, jedoch nicht zum Sieden kam (eventuell Bildung einer Suspension, bei erhöhter Temperatur jedoch klare Lösung). Es wurde zum Rückfluss erhitzt und bei dieser Temperatur gerührt bis das Edukt vollständig abreagiert war (DC-Kontrolle; ca. 6 h). Man ließ auf Raumtemperatur abkühlen und gab 2 g Palladium auf Kohle (1 Gew.-% bezogen auf eingesetztes Keton) zu. Dann wurde bei 50°C unter kräftigem Rühren Wasserstoff eingeleitet. Nach beendeter Reaktion (DC-Kontrolle, ca. 4h) wurde die rötliche, klare Lösung vom Katalysator abfiltriert. Zur Isolierung des Produktes wurde das Lösemittel am Rotationsverdampfer im Vakuum abdestilliert.

Es wurden 193 g 1,1-Di-(2-pyridyl)-methylamin (91 %) in Form eines rot-braunen Öls erhalten.

### 2. Stufe:

390,0 g Dipyridylmethylamin (95 %ig, 2 mol) wurden bei Raumtemperatur mit 1168 g (4,2 mol) 5N Natronlauge versetzt. Unter Kühlung wurde eine zuvor hergestellte Lösung aus 360,8 g (2,2 mol) Picolylchloridhydrochlorid in 700 ml Wasser so zugegeben, dass eine Innentemperatur von 35°C nicht überschritten wurde. Es wurde 3 h nachgerührt und dann gleichermaßen nacheinander weitere 1168 g (4,2 mol) 5N Natronlauge und nochmals eine Lösung von 360,8 g (2,2 mol) Picolylchloridhydrochlorid in 700ml Wasser zugegeben. Anschließend wurde bei Raumtemperatur 3 Tage kräftig gerührt. Zur Isolierung wurde die Reaktionsmischung dreimal mit je 1 L Dichlormethan extrahiert. Der organische Extrakt wurde am Rotationsverdampfer vollständig eingeengt, wobei man 780 g zähflüssiges Rohprodukt erhalten wurden. Zur Reinigung wurden 1406 g HBF4-Lösung (50 %ig in Wasser) und 1018 ml gesättigte Kochsalzlösung unter Kühlung zugesetzt. Bei Raumtemperatur wurde 1 h nachgerührt, die ausgefallenen Kristalle dann abfiltriert, gewaschen und getrocknet.

Es wurde 987 g Fluoroboratsalz erhalten.

Das Salz wurde in Dichlormethan bei Raumtemperatur fein suspendiert. Dann wurden 7,9 I 5N Natronlauge zugegeben, und die Mischung 30 Minuten kräftig gerührt.

Zur Aufarbeitung wurden die Phasen getrennt und die wässrige Phase noch zweimal mit je 19,7 I Dichlormethan extrahiert. Die gesamten organischen Phasen wurden über Na₂SO₄ gründlich getrocknet, und das Lösemittel abdestilliert.

Es wurden 478g 1,1,1',1"-Tetra-(2-pyridyl)-trimethylamin (65 %) als gelbliches Öl erhalten.

### 3. Stufe:

511,8 g (1,39 mol) frisch freigesetztes 1,1,1',1"-Tetra-(2-pyridyl)-trimethylamin wurden in THF gelöst und die Lösung unter Argon auf -70°C abgekühlt. Die Zugabe von 1000 ml (1,6 M in Hexan =1,6 mol) BuLi das zuvor auf -40°C abgekühlt wurde, erfolgte so, dass die Innentemperatur nicht über -65°C stieg. Nach beendeter Zugabe wurde noch 30 Minuten nachgerührt. Nach Ablauf dieser Zeit wurden 177,6 g (1,54 mol) TMEDA (N,N,N,N-Tetramethylethylendiamin) so zudosiert, dass die Innentemperatur nicht mehr als 5°C anstieg, und es wurde 10 Minuten nachgerührt. Nun wurden 226,5 g Methyliodid (1,6 mol) auf die gleiche Weise zugegeben. Etwa 10 Minuten nach beendeter Zugabe hatte die Reaktionsmischung eine Temperatur von -30°C. Die Mischung wurde 6 h Stunden bei dieser Temperatur und dann bis zum nächsten Morgen bei 0°C gerührt.

Bei dieser Temperatur wurden 49,8 g Glaubersalz hinzugefügt und wiederum 30 Minuten gerührt.

Der gebildete Niederschlag wurde über eine Glasfritte abfiltriert, zweimal mit je 75 ml kaltem THF gewaschen und bei 45°C im Vakuum getrocknet.

Das erhaltene Rohprodukt wurde in 2,9 kg Dichlormethan suspendiert. Zu dieser Suspension wurden 8,5 L 5N Natronlauge gegeben und die Mischung kräftig gerührt. Die Phasen wurden getrennt, die organische Phase mit gesättigter NaCl-Lösung gewaschen, über Na₂SO₄ getrocknet und vollständig eingeengt.

Es wurden 494,6 g (68 %) 1',1"-Di(2-pyridyl)-1,1-Di(2-pyridyl)dimethylethylamin als naturweißer Feststoff erhalten.

### 4. Stufe: Herstellung eines Eisen(II)chloridkomplexes mit Ligand nach (I):

140 g (0,366 mol) 1',1"-Di(2-pyridyl)-1,1-di(2-pyridyl)dimethylamin wurden in 3L Methanol gelöst. Bei Raumtemperatur wurden 61,1 g (0,366 mol) FeCl₂ x 4H₂O wurden zugegeben und 3 h nachgerührt. Nach beendeter Reaktionszeit wurden im Vakuum 2,7L Lösemittel (90 %) abdestilliert. Die erhaltene Produktsuspension wurde dann mit 3L Essigsäureethylacetat ca. 2 h ausgerührt, der Niederschlag abfiltriert und mit weiteren 200 ml Essigsäureethylacetat gewaschen.

Das feuchte Produkt wurde bei 40°C im Vakuum getrocknet.

Es wurden 171 g Produkt (92 %) als kräftig roter Feststoff erhalten.

## Patentansprüche

1. Verfahren zur Herstellung von N,N-Bis(pyridin-2-yl-methyl)-1,1-bis(pyridin-2-yl)-1-aminoalkan-Verbindungen der Formel 1 sowie Metallkomplexsalzes, die diese Verbindungen der Formel 1 als Ligand enthalten, 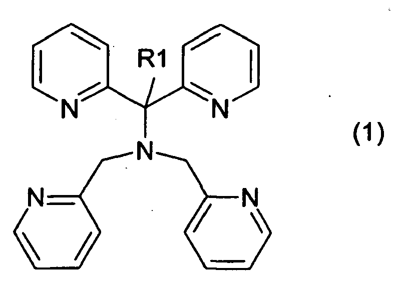 wobei R¹ C₁-C₆-Alkyl, Phenyl oder Benzyl, vorzugsweise Methyl bedeutet, **dadurch gekennzeichnet, dass** man
1) Dipyridylketon mit Hydroxylamin umsetzt, das so erhaltene Oxim ohne Zwischenisolierung zum Amin der Formel 2 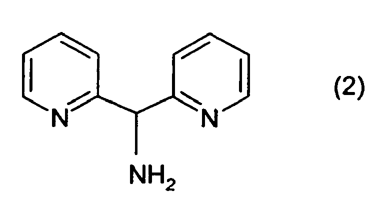 reduziert
2) das Amin der Formel 2 zunächst mit 1 bis 1,5 Moläquivalent Picolylchlorid und dann mit weiteren 1 bis 1,5 Moläquivalenten Picolylchlorid zum Amin der Formel 3 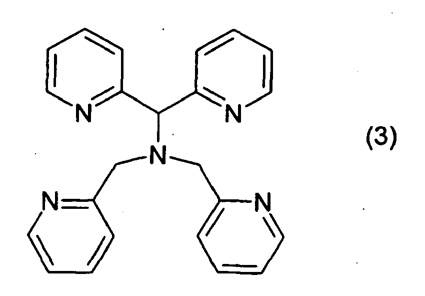 alkyliert,
3) das so erhaltene Amin der Formel 3 durch Zugabe von HBF₄ in das Fluoroborat-Salz überführt und anschließend das Amin der Formel 3 aus diesem Fluoroborat-Salz durch Zugabe einer Base freisetzt, und
4) das Amin der Formel 3 mit einem Alkylhalogenid der Formel R¹-hal alkyliert und gegebenenfalls
5) die so erhaltene Verbindung der Formel 1 durch Reaktion mit einem Übergangsmetallsalz in ein Übergangsmetallkomplexsalz überführt.

## Claims

1. A process for preparing N,N-bis(pyridin-2-ylmethyl)-1,1-bis(pyridin-2-yl)-1-aminoalkane compounds of the formula 1, and also metal complex salts containing these compounds of the formula 1 as ligand, 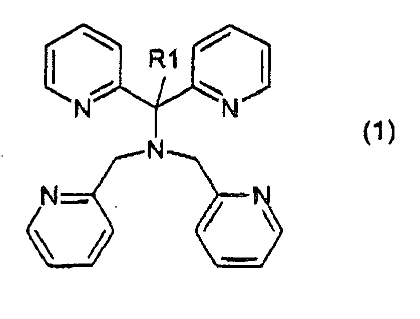 where R¹ is C₁-C₆ alkyl, phenyl or benzyl, preferably methyl, which comprises
1) reacting dipyridyl ketone with hydroxylamine and reducing the resulting oxime, without isolating it, to the amine of the formula 2 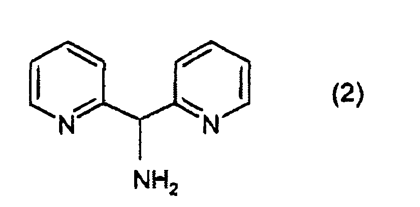
2) alkylating the amine of formula 2 first with 1 to 1.5 mole equivalents of picolyl chloride and then with a further 1 to 1.5 mole equivalents of picolyl chloride, to give the amine of the formula 3 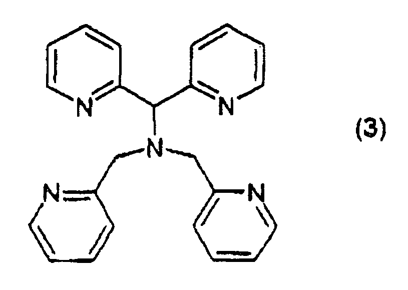
3) converting the resulting amine of formula 3 into the fluoroborate salt by addition of HBF₄, and then liberating the amine of formula 3 from this fluoroborate salt by addition of a base,and
4) alkylating the amine of formula 3 with an alkyl halide of the formula R¹-hal, and, if desired,
5) converting the resulting compound of formula 1 into a transition metal complex salt, by reaction with a transition metal salt.

## Revendications

1. Procédé de préparation de composés N,N-bis(pyridin-2-yl-méthyl)-1,1-bis(pyridin-2-yl)-1-aminoalcane de formule 1 ainsi que de sels de complexes métalliques qui renferment ces composés de formule 1 en tant que ligand, 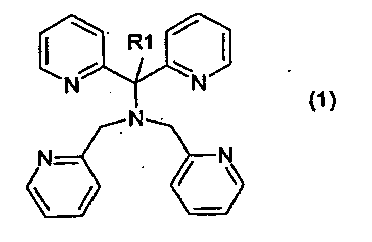 dans laquelle R¹ représente un groupe alkyle en C₁-C₆, un groupe phényle ou un groupe benzyle, de préférence un groupe méthyle, **caractérisé en ce que**
1) de la dipyridylcétone est mise à réagir avec de l'hydroxylamine, et l'oxime ainsi obtenu est réduit en l'amine de formule 2 sans isolation intermédiaire 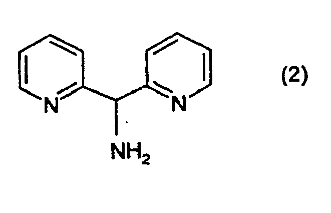
2) l'amine de formule 2 est d'abord alkylée avec de 1 à 1,5 équivalent molaire de chlorure de picolyle, puis avec de 1 à 1,5 équivalent molaire supplémentaire de chlorure de picolyle pour donner lieu à l'amine de formule 3 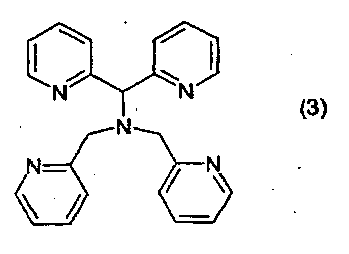
3) l'amine de formule 3 ainsi obtenue est transformée en le sel de fluoroborate par l'addition de HBF₄ et l'amine de formule 3 est ensuite libérée de ce sel de fluoroborate par l'addition d'une base, et
4) l'amine de formule 3 est alkylée avec un halogénure d'alkyle de formule R¹-hal, et éventuellement
5) le composé de formule 1 ainsi obtenu est transformé en un sel de complexe de métal de transition par réaction avec un sel de métal de transition.
